(19) Europäisches Patentamt
European Patent Office
Office européen des brevets

(11) **EP 4 763 064 A1**

(12) **EUROPEAN PATENT APPLICATION**

(43) Date of publication:
**24.06.2026 Bulletin 2026/26**

(21) Application number: **24221640.6**

(22) Date of filing: **19.12.2024**

(51) International Patent Classification (IPC):
**A61B 5/024** (2006.01)   **A61B 5/026** (2006.01)
**G02B 27/48** (2006.01)

(52) Cooperative Patent Classification (CPC):
**G02B 27/48; A61B 5/0077; A61B 5/02416;**
**A61B 5/0261; A61B 5/7207**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Stichting IMEC Nederland**
**5656 AE Eindhoven (NL)**

(72) Inventors:
• **LORATO, Ilde Rosa**
  **5653 JG Eindhoven (NL)**
• **HERMELING, Evelien**
  **6027 NT Soerendonk (NL)**
• **HERRANZ OLAZABAL, Jorge**
  **5595 HB Leende (NL)**

(74) Representative: **AWA Sweden AB**
**Matrosgatan 1**
**Box 5117**
**200 71 Malmö (SE)**

(54) **A METHOD AND A SYSTEM FOR REDUCING MOTION ARTEFACTS IN SPECKLE PATTERN SIGNAL**

(57)   A method for reducing motion artefacts in a speckle pattern signal comprises: illuminating (202) a region of a living being (10) by a light signal to form a speckle pattern; acquiring (204) a sequence of images of the region (12) of the living being (10); extracting (206) a speckle pattern signal from the acquired sequence of images to form a wave propagation signal representing a variation in time of a pressure wave or volumetric flow wave in the living being (10); acquiring (208) a motion compensation signal based on an optical measurement representing the region (12) of the living being (10), wherein the motion compensation signal represents a variation in time of motion of the living being (10); and performing (210) motion compensation of the wave propagation signal based on the motion compensation signal.

Fig. 2

**Description**

Technical field

The present description relates to detection of a speckle pattern from a living being, wherein a speckle pattern signal provides information relating to the living being. In particular, the present description relates to reducing motion artefacts in the speckle pattern signal.

Background

Speckles are interference patterns generated when a coherent light source, such as a laser, is used to illuminate a surface. Although often regarded as noise, speckles can also provide useful information. When the illuminated surface is static the speckle pattern will be static and form bright and dark dots. However, if the surface presents changes in time, such as movements or scattering changes, the speckle pattern will be perturbated.

Speckle patterns may be used for detecting a flow or a wave propagation in a living being. This may for instance be used for detecting blood flow in a person. Thus, a speckle pattern signal acquired from images of the speckle pattern may be used for determining a time series similar to a photoplethysmography (PPG) signal. This may be achieved by analyzing how the speckle contrast and/or other properties of the speckle pattern in the images changes in time.

However, monitoring a speckle pattern signal may be challenging in dynamic environments, such as for monitoring a speckle pattern of a person in a cabin environment. In such dynamic environments, there may be a relative movement between a sensor and the person, which may be caused by vehicle movements. This implies that motion artefacts may be common in the acquired speckle pattern signal such that information carried by the speckle pattern signal cannot be reliably determined.

Hence, there is a need for reducing motion artefacts in a speckle pattern signal.

Summary

An objective of the present description is to provide reduction of motion artefacts in a speckle pattern signal. Another objective is to provide reduction of motion artefacts in a simple manner providing a user-friendly arrangement for monitoring the speckle pattern signal.

These and other objectives are at least partly met by the invention as defined in the independent claims. Preferred embodiments are set out in the dependent claims.

According to a first aspect, there is provided a method for reducing motion artefacts in a speckle pattern signal, said method comprising: illuminating a region of a living being by a light signal to form a speckle pattern; acquiring a sequence of images of the region of the living being; extracting a speckle pattern signal from the acquired sequence of images to form a wave propagation signal representing a variation in time of a pressure wave or volumetric flow wave in the living being; acquiring a motion compensation signal based on an optical measurement representing the region of the living being, wherein the motion compensation signal represents a variation in time of motion of the living being; and performing motion compensation of the wave propagation signal based on the motion compensation signal.

According to the method, motion artefact reduction in a speckle pattern signal is provided in a simple manner. In particular, when the method is used for acquiring a speckle pattern signal from a human being, the method is user-friendly requiring no adaptation by a person from which the speckle pattern signal is to be acquired for enabling the motion artefact reduction.

Thus, the method may be particularly useful for implementation in a setting for acquiring a speckle pattern signal from a person during daily life activities of the person. For instance, the method may be implemented in a cabin environment, wherein a speckle pattern signal is acquired from a person in the cabin environment, such as from a person traveling with a vehicle. This implies that the speckle pattern signal may be acquired for monitoring health of the person without need of any adaptation by the person to allow the health monitoring to be performed. Rather, the speckle pattern signal may be acquired by providing illumination of a location in which the person will be situated and acquiring of images of the location. Further, the motion compensation signal may be acquired based on an optical measurement which does also not require any adaptation by the person.

The method may be particularly useful form implementation in a cabin environment, wherein motion artefacts may be very common due to a motion of a vehicle, such that motion artefact reduction may greatly improve signal-to-noise ratio in a motion compensated signal.

By the term "speckles" is here meant an interference pattern formed when coherent light illuminates a surface. On a static surface, the speckle pattern will be static and form bright and dark dots due to constructive and destructive interference of light. Speckle patterns may be formed when illuminating light from a light source is reflected by the surface, which may be referred to as reflection mode. Further, speckle patterns may be formed when illuminating light from the light source is transmitted through the surface, which may be referred to as transmission mode. In the present method, illuminating light may typically be reflected by the surface such that speckles are detected in reflection mode. However, it should be realized that light illuminating a surface of the living may exit at an opposite side of the living being, where a speckle pattern may be formed.

Living beings present movements of tissue, caused by for example blood flow through the tissue and contraction and dilatation of blood vessels in the tissue. In plants, movements of tissue may be caused by flow of sap through the plant. When living beings are illuminated by coherent light, the speckle pattern is perturbed by this movement, such that the speckle pattern is blurred, which may result in reduced speckle contrast of the speckle pattern.

It should be realized that flow in the tissue may be seen as a pressure wave or a volumetric flow wave propagating through vessels in tissue. The speckle pattern signal may represent the wave propagation based on the movement of the volumetric flow through the tissue and/or the contraction and dilation of vessels in the tissue affecting the speckle pattern. Thus, the speckle pattern signal may represent changes in a speckle pattern caused by wave propagation in the tissue. The signal representing wave propagation may thus be referred to as a wave propagation signal.

In comparison, in conventional Photo Plethysmography (PPG), not involving speckles, contraction and dilatation of blood vessels as the blood flows through the blood vessels is measured. PPG thus performs a measurement of volume change. Similar measurements performed using speckle pattern may be referred to Speckle Plethysmography (SPG), wherein a characteristic of the speckle pattern is measured. However, the SPG measurement may be affected also by the movements caused by the blood flow.

The wave propagation signal based on the speckle pattern signal used herein may be referred to as an SPG signal. However, although the term SPG implies measurements of volume change, it should be understood that an SPG signal does not necessarily result solely from volume change but may additionally or alternatively result from other changes of properties such as movements of the tissue and/or changes of scattering properties.

Speckle pattern changes from a living being may provide useful information in several applications, such as monitoring of vital signs of a person or an animal. Thus, the wave propagation signal after motion compensation may be used for monitoring of vital signs. The wave propagation signal may represent blood flow in the person or the animal.

By way of example, this could be used in medical applications. However, it should be understood that monitoring of vital signs may also be used in non-medical applications, such as in applications related to monitoring health of a driver and/or a passenger in a vehicle. Given as non-limiting examples, monitoring changes to a wave propagation signal may provide information on heart rate (HR), heart rate variability (HRV), pulse oximetry, blood pressure, vascular distensibility/compliance, pulse arrival time, pulse transit time, pulse wave velocity, blood flow velocity, capillary function, capillary refill time, capillary pressure, respiration rate, perfusion, and blood pressure.

By the term "light signal" is here meant any light signal that can form a speckle pattern. By way of example, the light signal may be a laser signal that may be formed by a laser, or a laser diode. However, the light signal may alternatively relate to a partially coherent light signal, such as a signal formed by passing light through a pinhole. Light for forming the light signal may thus be formed, e.g., by a light emitting diode (LED) or a superluminescent diode (SLD). In this context the term "light signal" is not limited to visible electromagnetic radiation. Rather, the term "light signal" may also include for example ultra-violet light and infra-red light. Preferably, the light signal may not relate to visible light such that illumination of the living being by the light signal is not noticeable to the living being.

The sequence of images may be acquired by any image sensor, such as a camera. Each image represents spatially resolved information of the region in which the speckle pattern is formed. The sequence of images may be formed by repetitively acquiring images of a region being monitored. The images may be acquired with a suitable rate for allowing information in the speckle pattern signal to be properly extracted. For instance, the sequence of images may be acquired with a frame rate of at least 10 Hz, such as at least 50 Hz, such as at least 100 Hz. However, it should be realized that substantially larger frame rates may also be used, such as a frame rate in order of kHz, such as up to, but not limited to, 100 kHz. The sequence of images may be a time sequence of images of a same region.

The region of the living being may cover the entire living being. However, according to embodiments, the region of the living being may be only part of the living being. For instance, if the method is used for acquiring a speckle pattern signal from a person, the region of the person may be a part of the person which is typically not covered by clothing to allow the speckle pattern signal to be affected by wave propagation in the person. Thus, the region may be a face and/or neck of the person. However, it should be realized that other regions may be used, depending on application. The entire or a part of the region of the living being may be illuminated by the light signal.

The motion compensation signal is acquired based on an optical measurement representing the region being illuminated by the light signal to form the speckle pattern. The optical measurement may be performed based on the entire region of the living being or on a part of the region. Thanks to using the same region of the living being for extracting the speckle pattern signal and for acquiring the motion compensation signal, the motion compensation is determined for motions affecting the speckle pattern signal. For instance, motion of a face of a person may be connected to motion of a neck of the person. Thus, the region may be a facial-cervical region and a motion compensation signal from the face of the facial-cervical region while the speckle pattern signal is extracted from the neck of the facial-cervical region. Similarly, a motion compensation signal from a wrist may be used for motion compensation of a speckle pattern signal acquired from a forearm of a same arm.

The motion compensation signal is acquired based on an optical measurement of the region of the living being. The motion compensation signal may be acquired in many different manners. The motion compensation signal may be acquired using a speckle pattern in a sequence of images of the region, such as the same sequence of images from which the speckle pattern signal is extracted. This may be a simple manner of acquiring the motion compensation signal since there may not be a need for separate hardware for acquiring the motion compensation signal and processing of the motion compensation signal may be similar to the processing of the speckle pattern signal.

However, it should be realized that the optical measurement may be performed in other manners. For instance, the motion compensation signal may be determined by image processing of a sequence of images. This may involve following movement of a particular feature, such as a facial landmark, in the sequence of images. Further, the motion compensation signal may be determined without necessarily acquiring images of the region. For instance, the motion compensation signal may be acquired as a photoplethysmography (PPG) signal representing an optical property of the tissue, such as reflectance or transmittance, as a variation in time. The PPG signal may also be affected by motion artefacts and may then be used for motion compensation of the wave propagation signal.

The motion compensation signal may thus be used for motion compensation of the wave propagation signal such that a motion compensated wave propagation signal is formed.

According to an embodiment, the speckle pattern signal is a first speckle pattern signal, and wherein acquiring of the motion compensation signal is based on determining a second speckle pattern signal representing the speckle pattern to form the motion compensation signal.

Thus, both the wave propagation signal and the motion compensation signal are determined based on speckle patterns. This may facilitate motion compensation since motion artefacts may be represented in a corresponding manner in the wave propagation signal and in the motion compensation signal. Hence, the method may be configured to significantly reduce motion artefacts, e.g., by completely or substantially removing motion artefacts from the wave propagation signal.

The first speckle pattern signal may be different from the second speckle pattern signal such that effects of motion artefacts may be reduced or removed from the wave propagation signal while information relating to propagation of the pressure wave or volumetric flow wave may be retained in the wave propagation signal.

According to an embodiment, the first speckle pattern signal is extracted from a first portion of the region and the second speckle pattern signal is determined from a second portion of the region, wherein the second portion is different from the first portion.

Thus, different portions of the region of the living being may be used for acquiring the first speckle pattern signal and the second speckle pattern signal. This implies that the wave propagation signal and the motion compensation signal may provide different information such that motion compensation may be efficiently performed.

A ratio of magnitude between information relating to wave propagation and information relating to motion artefacts may be different in the first and second speckle pattern signals. Thus, information relating to motion artefacts may be removed from or reduced in the wave propagation signal without information relating to wave propagation being removed.

For instance, the second speckle pattern signal may be acquired from a second portion in which no wave propagation information is present or in which wave propagation information has a smaller amplitude compared to the first portion. The second speckle pattern signal may be acquired from a second portion corresponding to a forehead of a person while the first speckle pattern signal may be acquired from a first portion corresponding to a neck of a person. Information of wave propagation of blood flow may not be prominent in the second speckle pattern signal since blood flow in forehead may only to a small extent affect a speckle pattern on the forehead, while movement due to motion artefacts in the second speckle pattern signal may be corresponding to movement due to motion artefacts in the first speckle pattern signal. Hence, the second speckle pattern signal may only or mostly carry information relating to motion artefacts allowing the motion compensation signal based on the second speckle pattern signal to efficiently provide motion compensation of the wave propagation signal.

It should be realized that the first portion and the second portion may be completely separate or may at least partially overlap. In some embodiments, the first portion may be a part of the second portion. For instance, the second portion may correspond to the entire region of the living being that is imaged in the sequence of images and the first portion may be a part of the region in which wave propagation is strongly represented in the speckle pattern signal.

According to an embodiment, the method further comprises identifying the first portion of the region in the sequence of images and the second portion of the region in the sequence of images based on identifying landmark features in the sequence of images, for using the first portion of the region in the extracting of the first speckle pattern signal and for using the second portion of the region in the determining of the second speckle pattern signal.

Thus, landmark features may be used for allowing accurate determination of the first portion and the second portion to be used for determining the wave propagation signal and the motion compensation signal, respectively. This ensures that the living being need not be placed in an exact position in relation to an image sensor for acquiring images. Rather, appropriate portions of the region of the living being for determining the wave propagation signal and the motion compensation signal may still be identified in the sequence of images.

The landmark features may for instance correspond to features of a facial-cervical region for identifying portions in the facial-cervical region. For instance, the landmark features may comprise a nose tip or a chin.

This may facilitate automatic identification of portions to be used for determining the wave propagation signal and the motion compensation signal, respectively.

The first and second portions of the region may be determined from the same sequence of images. However, according to an alternative, the first and second portions of the region may be determined from different sequences of images acquired by different image sensors that image different parts of the region.

According to an embodiment, acquiring of the motion compensation signal comprises determining one or more further speckle pattern signals from respective further portions of the region.

This may facilitate using different motion compensations at different points in time. Thus, the method may not need to perform a static processing of signals such that motion compensation is always to be performed from a particular portion. Rather, the method may allow the motion compensation to be dynamically controlled such that an efficient motion compensation may be achieved.

According to an embodiment, the motion compensation signal is determined from a combination of the second speckle pattern signal and the one or more further speckle pattern signal.

An efficient motion compensation may be provided by using information from a combination of portions. The portions of the region may not necessarily form a coherent part of the region.

According to an embodiment, the motion compensation signal is determined from a dynamic selection from the second speckle pattern signal and the one or more further speckle pattern signal.

Thus, the portion to be used for determining the motion compensation signal may be dynamically changed. This implies that the method may ensure that an efficient motion compensation is acquired by selecting the second or further speckle pattern signal to be used for motion compensation that at a particular point in time provides efficient motion artefact reduction.

The method may be configured to evaluate which of the second speckle pattern signal or the one or more further speckle pattern signals to be used at a regular time intervals. Alternatively, the method may be configured to update the dynamic selection upon identifying that signal-to-noise ratio of the wave propagation signal decreases below a threshold.

According to an embodiment, the first speckle pattern signal and the second speckle pattern signal are extracted from the sequence of images.

This implies that a single sequence of images may be acquired. This may facilitate motion compensation since there is only need for a single image sensor for determining the wave propagation signal and the motion compensation signal.

According to an alternative, the first speckle pattern signal and the second speckle pattern signal are extracted from sequences of images acquired by different image sensors. Thus, two different image sensors, such as two different cameras, may be used for acquiring the first speckle pattern signal and the second speckle pattern signal, respectively.

According to an embodiment, the first speckle pattern signal is based on a representation of the speckle pattern with a first lens parameter and the second speckle pattern signal is based on a representation of the speckle pattern with a second lens parameter.

Thus, the first speckle pattern signal and the second speckle pattern signal may be extracted from a same portion of the region although the portion of the region is represented by different lens parameters. Using different lens parameters, the information of wave propagation may be more prominent in the first speckle pattern signal, such that motion compensation may be performed based on the second speckle pattern signal.

It should also be realized that the first speckle pattern signal and the second speckle pattern signal may be acquired using a common imaging system, such as a single camera allowing different lens parameters to be used simultaneously within the single camera. This may be achieved using an array of microlenses.

Alternatively, the first speckle pattern signal and the second speckle pattern signal may be acquired using different image sensors providing different lens parameters. The first speckle pattern signal and the second speckle pattern signal may also or alternatively be acquired from different portions of the region of the living being.

According to an embodiment, the first speckle pattern signal is based on a representation of the speckle pattern with a first magnification and the second speckle pattern signal is based on a representation of the speckle pattern with a second magnification.

Thus, the first speckle pattern signal and the second speckle pattern signal being acquired with different magnifications is an example of the speckle pattern signals being acquired using different lens parameters. Using different magnifications may be a suitable manner of ensuring that information of wave propagation may be more prominent in the first speckle pattern signal, such that motion compensation may be performed based on the second speckle pattern signal.

According to an alternative, the lens parameters may instead or additionally be a size of a lens aperture.

According to an embodiment, performing motion compensation comprises forming a linear combination of the wave propagation signal and the motion compensation signal.

Thus, a simple processing may be performed for providing motion compensation of the wave propagation signal. Thus, processing for motion compensation may be performed quickly requiring limited computer resources.

For instance, motion compensation may be achieved by subtracting the motion compensation signal from the wave propagation signal. This may be a very simple operation such that motion compensation may be very quickly performed.

However, it should be realized that other combinations of the wave propagation signal and the motion compensation signal may be used for providing motion compensation. For instance, blind source separation (BSS), independent component analysis (ICA), principal component analysis (PCA) or linear combinations in frequency domain of the wave propagation signal and the motion compensation signal may be used.

According to an embodiment, the living being is a human being and the region illuminated by the light signal is at least part of a facial-cervical region.

This may be particularly useful for providing monitoring of a person using a speckle pattern signal without affecting the person. For instance, no adaptation may be needed in order for the acquiring of the speckle pattern signal with motion compensation to be enabled.

In particular, a strong signal dependent of blood flow may be acquired from a neck of the person, such that the facial-cervical portion may facilitate acquiring of a wave propagation signal carrying information of blood flow. Further, a face portion may be suitable for providing motion compensation information.

According to an embodiment, acquiring the motion compensation signal comprises acquiring a light intensity signal representing a variation in time of absorption or reflection of light by a volumetric flow in the living being.

Thus, the motion compensation signal may be acquired by acquiring a photoplethysmogram (PPG) signal. The PPG signal may be acquired by illuminating a region of the living being by a light signal and measuring an intensity of light transmitted through a volumetric flow in tissue of the living being or reflected by the volumetric flow in tissue of the living being. The PPG signal may be acquired using a different light signal to the light signal used for acquiring the speckle pattern signal.

The PPG signal may be affected by motion artefacts enabling the PPG signal to be used for motion compensation of the wave propagation signal formed by extracting the speckle pattern signal from the sequence of images.

The PPG signal may be relatively simple to acquire since it may not require imaging. Rather, it may be sufficient to measure a light intensity in a single position.

According to an embodiment, acquiring the motion compensation signal comprises identifying a landmark feature in a sequence of images, and determining movements of the landmark feature between images in the sequence of images to form a representation of motion of the living being.

The landmark features may be easily identifiable features in the living being. For example, for determining a wave propagation signal in a human being, the landmark feature may be a nose tip or an eye. The motion compensation signal may be determined based on movements of one or more landmark features.

The motion compensation signal may be acquired using the same sequence of images that is also used for extracting the speckle pattern signal. This implies that the method may be performed with few components.

However, it should be realized that the motion compensation signal may be determined using a separate sequence of images.

According to a second aspect, there is provided a computer program product, comprising computer-readable instructions which when executed by a processing unit cause the processing unit to perform a method for reducing motion artefacts in a speckle pattern signal, said method comprising: receiving a sequence of images of the region of the living being; extracting a speckle pattern signal from the acquired sequence of images to form a wave propagation signal representing a variation in time of a pressure wave or volumetric flow wave in the living being; receiving a motion compensation signal based on an optical measurement representing the region of the living being, wherein the motion compensation signal represents a variation in time of motion of the living being; and performing motion compensation of the wave propagation signal based on the motion compensation signal.

Effects and features of this second aspect are largely analogous to those described above in connection with the first aspect. Embodiments mentioned in relation to the first aspect are largely compatible with the second aspect.

The computer program product may thus provide computer-readable instructions for allowing a method to be implemented for performing processing of the speckle pattern signal to provide motion artefact reduction.

The computer program product may comprise a non-transient computer-readable medium for carrying the computer-readable instructions. Alternatively, in other embodiments, the computer program product may comprise a signal carrying the computer-readable instructions, e.g., for communicating the computer program product to the processing unit through wired or wireless communication.

According to a third aspect, there is provided a system for reducing motion artefacts in a speckle pattern signal, the system comprising: a light source configured to illuminate a region of a living being by a light signal for forming a speckle pattern; an imaging unit configured to acquire a sequence of images representing the speckle pattern at the region of the living being; a processor configured to: receive the sequence of images from the imaging unit; extract a speckle pattern signal from the received sequence of images to form a wave propagation signal representing a variation in time of a pressure wave or a volumetric flow wave in the living being; receive a motion compensation signal based on an optical measurement representing the region of the living being, wherein the motion compensation signal represents a variation in time of motion of the living being; and perform motion compensation of the wave propagation signal based on the motion compensation signal.

Effects and features of this third aspect are largely analogous to those described above in connection with the first and second aspects. Embodiments mentioned in relation to the first and second aspects are largely compatible with the third aspect.

According to the system, motion artefact reduction in a speckle pattern signal is provided in a simple manner. In particular, when the system is used for acquiring a speckle pattern signal from a human being, the system is user-friendly requiring no adaptation by a person from which the speckle pattern signal is to be acquired for enabling the motion artefact reduction.

By the term "light source" is here meant any unit, device and/or element at which the light for forming the light signal that forms a speckle pattern is generated. By way of example, the light source may be, but is not limited to a laser, or a laser diode. However, the light source may alternatively be a LED or SLD for generating at least partially coherent light.

The imaging unit may be any device configured to form an image of the speckle pattern. The imaging unit may be configured to form a two-dimensional representation of light intensity of the speckle pattern. The imaging unit may comprise a lens configured to form an image onto an array of photo-sensitive elements. Given as non-limiting examples, photo-sensitive elements may be photodiodes or a photo-multiplier tubes (PMT). Given as further non-limiting examples, the photo-sensitive elements may be formed by an image sensor such as a charge-coupled device (CCD), a complementary metal oxide semiconductor (CMOS), or indium gallium arsenide (InGaAs) sensor. For example, the imaging unit may be formed by a camera.

It should be realized that the system may comprise a plurality of light sources and/or a plurality of imaging units. This implies that different light sources may be used for illuminating different portions of the region of the living being. Similarly, different imaging units may be used for acquiring separate sequences of images of different portions of the region.

The processor may be implemented as any processing unit, such as a general-purpose processing unit, e.g., a central processing unit (CPU), which may execute instructions of one or more computer programs in order to implement functionality of the processor. The processor may also or alternatively be implemented as firmware arranged e.g., in an embedded system, or as a specifically designed processing unit, such as an Application-Specific Integrated Circuit (ASIC) or a Field-Programmable Gate Array (FPGA). It should be realized that the processor may be implemented as a combination of hardware and software components.

All parts of the system may be mounted in a common housing. This may ensure proper arrangement of the light source and the imaging unit in relation to the living being to be monitored. Further, the processor may be arranged in the same housing as the light source and the imaging unit. This may ensure that a compact system is provided, allowing assessment of the speckle pattern signal locally where measurements on a living being is performed.

However, it should be realized that the processor may be arranged in a different physical location and/or may be distributed between different physical processing units. Thus, the processor may be provided anywhere, such as "in the cloud". The processor may communicate with the imaging unit through a computer network, such as the Internet, enabling the processor to be arranged anywhere in relation to the imaging unit for receiving the sequence of images.

According to an embodiment, the processor is configured to acquire the motion compensation signal based on processing of the received sequence of images.

Thus, a single sequence of images may be acquired for providing motion artefact reduction. This implies that a single imaging unit may be used such that the system is simple requiring few components.

This implies that the processor is configured to receive the motion compensation signal as part of receiving the sequence of images and may extract the motion compensation signal from the sequence of images.

According to an embodiment, the imaging unit is configured to acquire one or more sequences of images representing the speckle pattern using different lens parameters, wherein the processor is configured to extract the speckle pattern signal and acquire the motion compensation signal based on the different lens parameters.

For instance, the different lens parameters may be different magnifications. Different magnifications of the same speckle pattern may allow motion compensation to be determined. The different magnifications may be acquired using a single imaging unit such that a single sequence of images is acquired or using at least two imaging units for acquiring sequences of images with different magnifications.

According to a fourth aspect, there is provided a vehicle comprising: a system according to the third aspect, wherein the system is mounted in the vehicle for illuminating the living being that is a human being traveling with the vehicle, wherein the system is configured to determine a motion compensated wave propagation signal compensating for motion artefacts due to movement of the vehicle.

Effects and features of this fourth aspect are largely analogous to those described above in connection with the first, second, and third aspects. Embodiments mentioned in relation to the first, second, and third aspects are largely compatible with the fourth aspect.

The system of the third aspect may be particularly useful for monitoring a human being traveling with a vehicle. Motion artefacts may be frequently caused by vehicle movement, e.g., due to irregularities on a road, speed bumps, acceleration, and deceleration. Thanks to providing reduction of motion artefacts, effects of the vehicle movements on quality of the wave propagation signal may be avoided.

It should further be realized that the system may be used in other applications.

According to another aspect, the system is configured to be mounted in a machine environment for illuminating the living being that is a human being present in the machine environment, wherein the system is configured to determine a motion compensated wave propagation signal compensating for motion artefacts due to movement in the machine environment.

The machine environment may be any environment wherein a man-machine interface is provided. Thus, the system may be configured to monitor health of a person present in the machine environment. This may be used for identifying any changes to the health of the person that may affect the ability of the person to control the machine environment. Such identification may be important in a vehicle to identify if a driver is unable to control the vehicle such that the moving vehicle may be automatically brought to a stop to avoid accidents. However, such identification may also be important to identify if an operator in a control room is unable to monitor or control machinery from the control room.

Brief description of the drawings

The above, as well as additional objects, features, and advantages of the present description, will be better understood through the following illustrative and non-limiting detailed description, with reference to the appended drawings. In the drawings like reference numerals will be used for like elements unless stated otherwise.

Fig. 1 is a schematic view of a general system for reducing motion artefacts in a speckle pattern signal.
Fig. 2 is a flow chart of a general method for reducing motion artefacts in a speckle pattern signal.
Fig. 3 is a flow chart relating to a method and a system according to a first embodiments.
Fig. 4 is a schematic view of time-varying signals illustrating motion compensation by the method of the first embodiment.
Fig. 5 is a schematic view of a system according to a second embodiment.
Fig. 6 is a schematic view of a system according to a third embodiment.
Fig. 7 is a schematic view of a system according to a fourth embodiment.
Fig. 8 is a schematic view of a vehicle according to an embodiment.

Detailed description

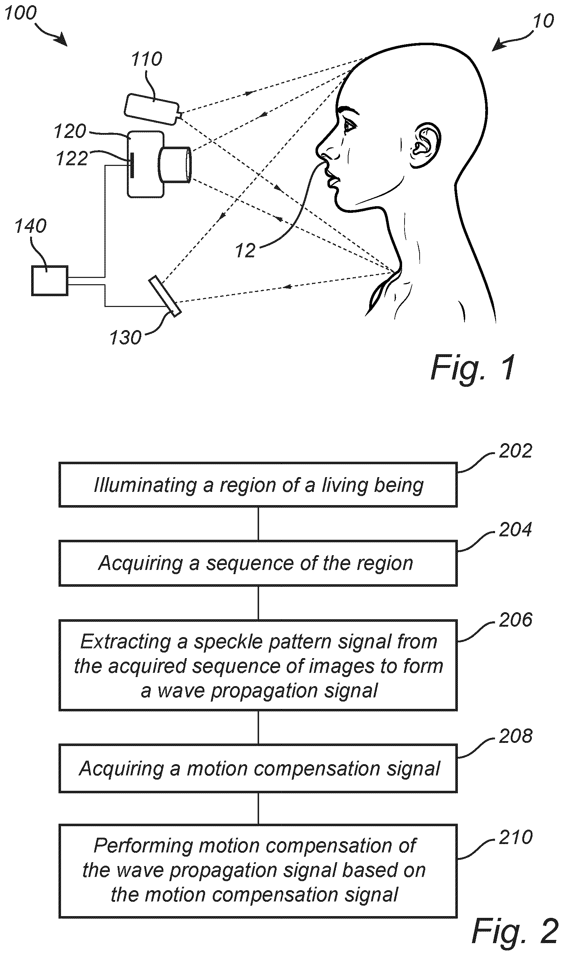

Referring now to Fig. 1, a system 100 for acquiring a speckle pattern signal and for reducing motion artefacts in the speckle pattern signal will be described. The system 100 will be described below in relation to acquiring a speckle pattern signal from a human being but it should be realized that the system 100 may alternatively be used for acquiring a speckle pattern signal from any living being, such as an animal or a plant.

The system 100 comprises a light source 110 configured to illuminate a region 12 of a person 10 by a light signal, such as a laser signal. Hereinafter, a laser signal generated by a laser source 10 will be discussed but it should be realized that as an alternative, the light signal may be an at least partially coherent light signal. The laser signal from the light source 110 illuminating the region 12 is scattered in the region 12 and forms a speckle pattern. For instance, the laser source 110 may be configured to illuminate a facial-cervical region 12 of the person 10. However, it should be realized that any other region of the person may be illuminated.

The laser source 110 may be associated with an optical component for forming a diverging beam of the laser signal such that a large region 12 is illuminated by the laser signal. However, the laser source 110 may have a relatively large output angle such that the region 12 is illuminated by the laser signal.

The laser source 110 may be configured to illuminate bare skin of the person 10 so as to allow the speckle pattern formed on skin to be affected by a volumetric flow in tissue of the region.

The speckle pattern may be affected by a pressure wave or a volumetric flow wave, which may be caused by circulation of blood in the person. It should be realized that the wave propagation may cause movement of walls of a vessel, such as contraction and dilation of vessels which may affect the speckle pattern on the skin by, for example, causing the speckle pattern on the skin to be affected. For instance, a size or location of speckles in the speckle pattern may be changed or a contrast of the speckle pattern may be changed.

The system 100 further comprises an imaging unit 120 configured to acquire a sequence of images representing the speckle pattern that is formed on the region of the living being, such as the speckle pattern formed on skin, e.g., in the facial-cervical region 12, of the person 10. The imaging unit 120 may configured to acquire a two-dimensional representation of the region. The imaging unit 120 may thus comprise an optical system for forming an image of the region onto an image sensor 122. The image sensor 122 may for instance comprise an array of photo-sensitive elements for detecting intensity of light in a location of respective photo-sensitive elements. The image sensor 122 may for instance be formed by a charge-coupled device (CCD) sensor or by a complementary metal-oxide-semiconductor (CMOS) sensor.

The sequence of images acquired by the imaging unit 120 may thus represent a speckle pattern signal. In each image, a value of a characteristic of the speckle pattern signal, such as size or location of speckles or contrast of the speckle pattern, may be determined. Thus, a sequence of values of the characteristic of the speckle pattern may be determined from the sequence of images forming a wave propagation signal representing a variation in time of the value of the characteristic of the speckle pattern. The wave propagation signal may further provide a representation of propagation of a pressure wave or a volumetric flow wave in the person 10 based on the pressure or volumetric flow wave affecting the characteristic of the speckle pattern on the region 12.

The laser source 110 and the imaging unit 120 may be mounted in relation to each other such that the imaging unit 120 is configured to image an area which is illuminated by the laser signal output by the laser source 110. Thus, the laser source 110 and the imaging unit 120 may be provided with a fixed relation such that the system 100 is prepared for monitoring of a speckle pattern.

The system 100 is further configured to determine a motion compensation signal, representing movements of the region 12 which are not due to wave propagation and therefore may affect accuracy of the wave propagation signal. The motion compensation signal may be determined by processing the sequence of images acquired by the imaging unit 120, i.e., the same sequence of images used for determining the wave propagation signal. However, the system 100 may alternatively comprise a separate motion artefact sensor 130 configured to detect movements so as to allow determining the motion compensation signal. The motion compensation signal may be determined based on an optical measurement representing the region 12 of the person 10 as will be described in further detail below for different embodiments.

The system 100 further comprises a processor 140. The processor may be implemented as any processing unit, such as a general-purpose processing unit, e.g., a central processing unit (CPU), which may execute instructions of one or more computer programs in order to implement functionality of the processor. The processor may also or alternatively be implemented as firmware arranged e.g., in an embedded system, or as a specifically designed processing unit, such as an Application-Specific Integrated Circuit (ASIC) or a Field-Programmable Gate Array (FPGA). It should be realized that the processor may be implemented as a combination of hardware and software components.

The processor 140 may be mounted together with the laser source 110 and/or the imaging unit 120, such as being arranged close to and directly connected to the imaging unit 120 for receiving the sequence of images. However, the processor 140 may be arranged in any other location and may be configured to communicate with the imaging unit 120 through wired and/or wireless communication. For instance, the processor 140 may be implemented by a server "in the cloud".

The processor 140 is configured to receive the sequence of images from the imaging unit 120. The processor is further configured to extract a speckle pattern signal from the received sequence of images to form a wave propagation signal representing a variation in time of a pressure wave or a volumetric flow wave in the region 12 of the person 10.

The processor 140 is further configured to receive a motion compensation signal, such as receiving a motion compensation signal from the motion artefact sensor 130. The motion compensation signal represents a variation in time of motion of the person 10, i.e., a relative motion of the person 10 in relation to the laser source 110 and/or the imaging unit 120.

The processor 140 is further configured to perform motion compensation of the wave propagation signal based on the motion compensation signal.

Measurements of the speckle pattern may be referred to as Speckle Plethysmography (SPG), wherein a characteristic of the speckle pattern is measured. The feature may for instance be size or location of speckles and/or contrast of the speckle pattern. The feature may represent dilation or contraction of vessels and, hence, the use of the term plethysmography. However, the SPG measurement may be affected also by the movements caused by volumetric flow through tissue. Nevertheless, a measurement performed on the speckle pattern may be referred to as a SPG signal.

Referring now to Fig. 2, a method for reducing motion artefacts in a speckle pattern signal will be described.

The method comprises illuminating 202 a region of a living being, such as the facial-cervical region 12 of a person 10, by a laser signal to form a speckle pattern.

The method further comprises acquiring 204 a sequence of images of the region 12 of the person 10 and extracting 206 a speckle pattern signal from the acquired sequence of images to form a wave propagation signal representing a variation in time of a pressure wave or volumetric flow wave in the person 10.

The method further comprises acquiring 208 a motion compensation signal based on an optical measurement representing the region 12 of the person 10, wherein the motion compensation signal represents a variation in time of motion of the person 10.

The motion compensation signal may represent only motion of the person 10 and may not provide any information relating to the wave propagation. However, the motion compensation signal may alternatively be determined based on a measurement that represents wave propagation and is also affected by motion of the person 10. A relation between ratio of wave propagation and motion artefacts, for instance, a signal-to-noise ratio, may be different in the wave propagation signal compared to the motion compensation signal. This may be utilized for removing or at least reducing impact of motion artefacts in the wave propagation signal while maintaining information relating to the wave propagation.

Thus, the method further comprises performing 210 motion compensation of the wave propagation signal based on the motion compensation signal. The motion compensation may be performed by forming a linear combination of the wave propagation signal and the motion compensation signal, such as by subtracting the motion compensation signal from the wave propagation signal.

However, it should be realized that other combinations of the wave propagation signal and the motion compensation signal may be used for providing motion compensation. For instance, blind source separation (BSS), independent component analysis (ICA), principal component analysis (PCA) or linear combinations in frequency domain of the wave propagation signal and the motion compensation signal may be used.

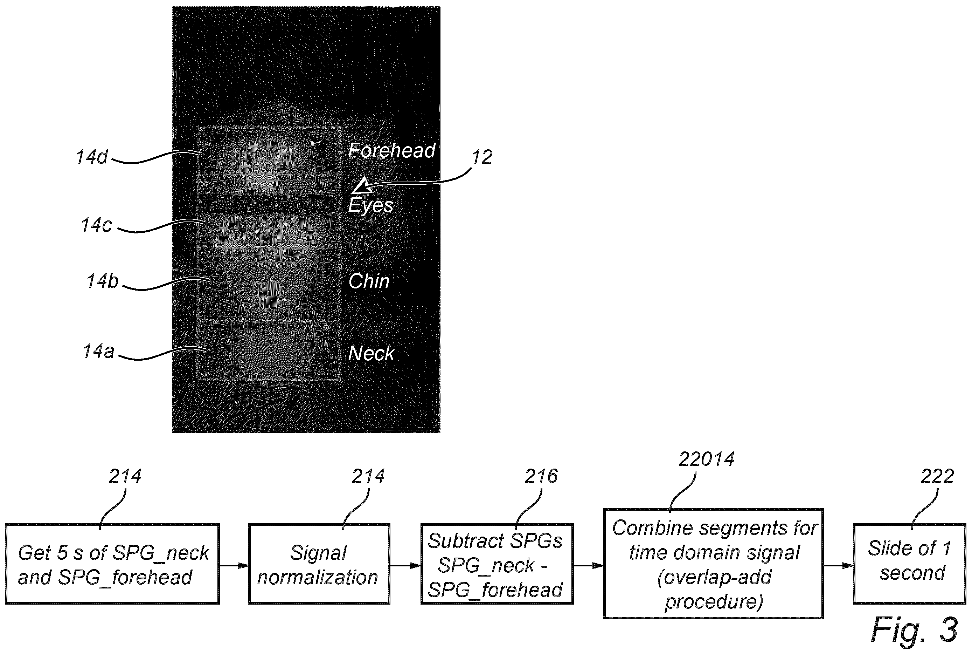

Referring now to Fig. 3, a method and system for motion artefact reduction according to a first embodiment will be described. The method may be performed by a system having no separate motion artefact sensor. Instead, the system is configured to process the sequence of images for determining the motion compensation signal from the sequence of images. Thus, the system may comprise a laser source and an imaging unit as described above, and a processor configured to receive the sequence of images for determining the wave propagation signal and the motion compensation signal. The processor may be configured to perform the method as described below.

In this method, a facial-cervical region 12 of the person is illuminated by the laser signal and a sequence of images of the facial-cervical region 12 is acquired by the imaging unit 120.

As indicated in Fig. 3, different portions 14a-d in an image of the facial-cervical region 12 may be defined. Each portion 14a-d may be considered to represent a separate speckle pattern signal. Thus, the speckle pattern in each portion 14a-d may be separately processed for determining a value of the characteristic of the speckle pattern in the portion 14a-d for forming a time-varying signal from the portion 14a-d in the sequence of images. Thus, separate speckle pattern signals may be extracted from the same sequence of images.

A first speckle pattern signal may be extracted for determining a first SPG signal representing wave propagation. The first SPG signal may be extracted from a first portion 14a of the facial-cervical region 12, e.g., a neck portion, wherein a strong effect of wave propagation may be visible in the SPG signal.

A second speckle pattern signal may be extracted for determining a second SPG signal representing motion compensation. The second SPG signal may be extracted from a second portion 14d of the facial-cervical region 12, being a different portion from the first portion 14a used for extracting the first SPG signal. For instance, the second SPG signal may be extracted from a forehead portion of the facial-cervical region 12.

The method may comprise identifying the first portion 14a of the region 12 in the sequence of images and the second portion 14d of the region 12 in the sequence of images based on identifying landmark features in the sequence of images. Thus, image processing using facial landmark algorithms may be used for identifying the portions 14a-d of the region 12. After identifying of the portions 14a-d of the region, the neck portion 14a may be used for extracting the first SPG signal and the forehead portion 14d may be used for extracting the second SPG signal.

The method for performing motion compensation may comprise selecting the SPG signal to be used for representing the wave propagation and the SPG signal to be used for representing motion compensation. Thus, the first SPG signal from the neck portion 14a may be selected for representing wave propagation and the second SPG signal from the forehead portion 14d may be selected for representing motion compensation.

As illustrated in Fig. 3, the method may comprise acquiring 214 a time segment of the first SPG signal and a corresponding time segment of the second SPG signal. Thus, the first SPG signal and the second SPG signal are acquired for a common period in time. The period in time may for instance have a duration of a few seconds, such as 5-10 seconds.

The period in time may form a time window based on which motion compensation is performed. The method may use a sliding time window such that time windows in a sequence of time windows are overlapping. This may enable motion artefacts to be efficiently removed or reduced.

The method may further comprise normalizing 216 the first SPG signal and the second SPG signal. This may facilitate correctly combining the signals for providing motion compensation.

The method may further comprise subtracting 218 the second SPG signal from the first SPG signal. Thus, the first SPG signal may be compensated such that motion artefacts may be reduced or removed from the first SPG signal.

The method may further comprise combining 220 the segment of the motion-compensated first SPG signal with previous segments in the sequence of time windows. This may allow a motion-compensated signal with a high signal-to-noise ratio to be determined.

Thereafter, the method may comprise sliding 222 the time window and repeating the steps 214-220 described above.

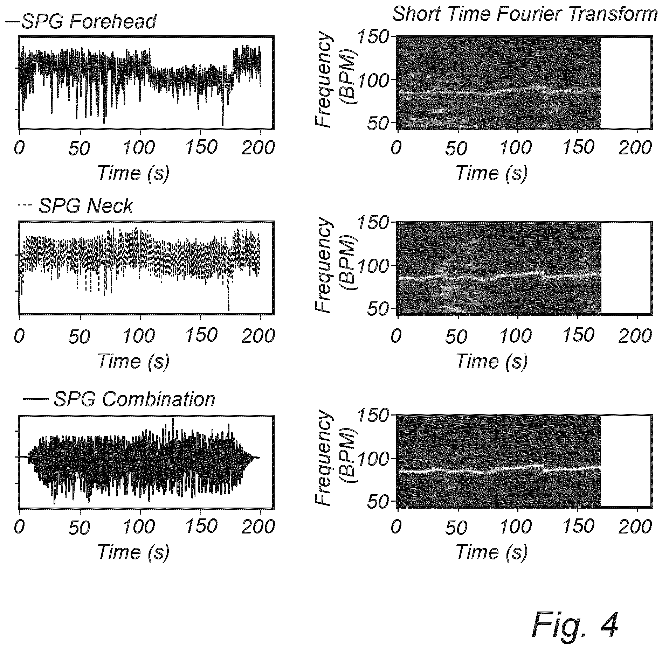

Referring now to Fig. 4, an example of results is illustrated. Fig. 4 shows at a left-hand side, different time domain SPG signals, and at a righthand side, corresponding frequency domain SPG signals acquired by using a short-time Fourier transform on the time domain SPG signal. In the top row of Fig. 4, a second SPG signal acquired from the forehead portion 14d is illustrated. In the middle row of Fig. 4, a first SPG signal acquired from the neck portion 14a is illustrated. In the bottom row of Fig. 4, a motion compensated SPG signal acquired by subtracting the second SPG signal from the first SPG signal is illustrated.

As shown in Fig. 4, frequency information relating to movements that do not correspond to wave propagation is significantly reduced in the motion compensated SPG signal.

The first SPG signal may be considered as a combination of wave propagation information and motion artefacts and may thus be represented as:

$$S1 = F1 + M1,$$

where S1 is the first SPG signal, F1 is the information relating to wave propagation (flow) in the first SPG signal and M1 is the information relating to movements, forming an additive component to the wave propagation information.

Similarly, the second SPG signal may be represented as:

$$S2 = F2 + M2,$$

where S2 is the second SPG signal, F2 is the information relating to wave propagation (flow) in the second SPG signal and M2 is the information relating to movements, forming an additive component to the wave propagation information. The information of movements to be compensated for may be represented in a similar manner in the first and the second SPG signal (having similar amplitude and phase).

Thus, the subtraction of the second SPG signal from the first SPG signal may be represented as:

$$S1 - S2 = F1 + M1 - (F2 + M2) \approx F1 - F2,$$

where M2 may remove the M1 component from the first SPG signal since the movement artefacts are represented in similar manner, whereas amplitude and/or phase of the information relating to wave propagation is different between the first SPG signal and the second SPG signal such that the wave propagation information is maintained after motion compensation.

Above, motion compensation has been described using the forehead portion 14d. However, it should be realized that acquiring of the motion compensation signal may comprise determining one or more further SPG signals from respective further portions of the region. Thus, in addition to the forehead portion 14d, a chin portion 14b and an eye portion 14c may also be used for determining further SPG signals.

The motion compensation signal may be determined from a combination of the second SPG signal and further SPG signals. This may provide strengthening the information relating to motion compensation in the motion compensation signal such that motion artefact reduction may be efficiently provided.

Alternatively, each of the SPG signals may be considered as a candidate for providing motion compensation. Thus, a signal to be used for motion compensation may be determined from a dynamic selection of the second SPG signal or a further SPG signal. The dynamic selection may for instance be determined based on a signal-to-noise ratio of a combination (e.g., through subtraction) of the first SPG signal with each of the second SPG signal and the further SPG signals, respectively. The combined signal having highest signal-to-noise ratio may then be used for providing a motion-compensated signal.

The dynamic selection may be based on an initial time segment and may then be maintained for a period of time, such as an entire session of monitoring the person 10.

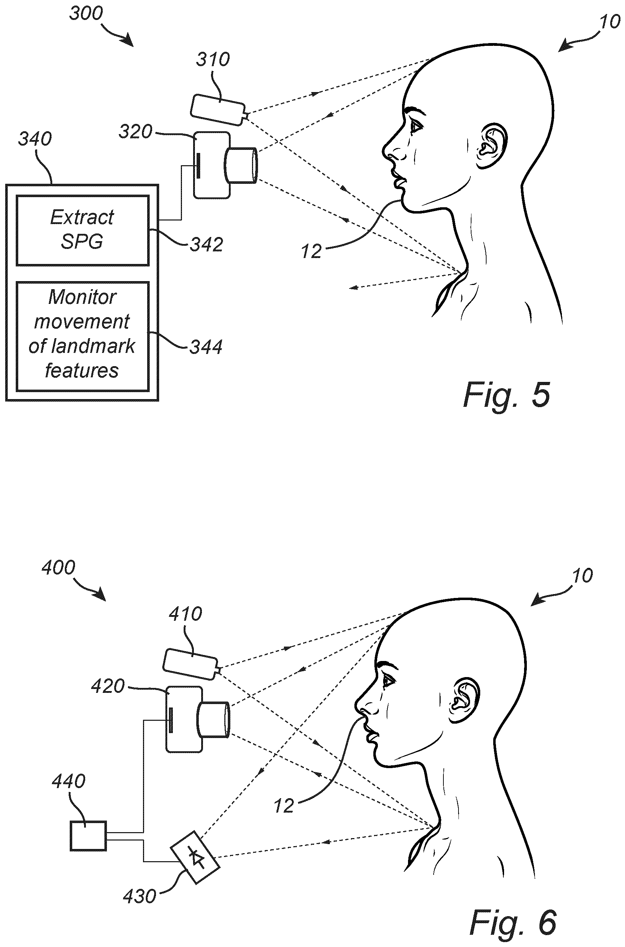

Referring now to Fig. 5, a method and system 300 for motion artefact reduction according to a second embodiment will be described. Like the system described in relation to Fig. 3 above, the system 300 may have no separate motion artefact sensor.

The system 300 is configured to process the sequence of images for determining the motion compensation signal from the sequence of images. Thus, the system 300 may comprise a laser source 310 and an imaging unit 320 as described above.

The system 300 may further comprise a processor 340 configured to receive the sequence of images for determining the wave propagation signal and the motion compensation signal. The wave propagation signal may be determined by extracting a SPG signal from the sequence of images, indicated by block 342 in the processor 340.

However, instead of extracting another SPG signal to be used for motion compensation, the processor 340 may be configured to perform image processing of the sequence of images for determining the motion compensation signal, indicated by block 344 in the processor 340.

A method for motion artefact reduction may thus comprise identifying a landmark feature in the sequence of images. The landmark feature may be a characteristic and easily identifiable feature in the region 12 of the person 10. Thus, the landmark feature may for instance be a nose tip of the person 10.

The method may further comprise determining movements of the landmark feature between images in the sequence of images. Thus, a spatial location of the landmark feature may be determined in each image in the sequence of images allowing movements of the landmark feature to be determined by comparing the spatial locations between sequential images in the sequence of images.

The method may thus comprise determining the motion compensation signal based on the determined movements of the landmark feature. This motion compensation signal may be used instead of the motion compensation signal based on SPG described above for providing motion compensation of the first SPG signal representing wave propagation.

Referring now to Fig. 6, a method and system 400 for motion artefact reduction according to a third embodiment will be described.

The system 400 may comprise a laser source 410 and an imaging unit 420 as described above. The system 400 may further comprise a motion artefact sensor 430. Thus, the system 400 may comprise a separate sensor for performing an optical measurement representing the region 12 of the person 10.

The motion artefact sensor 430 may be configured to determine a photoplethysmogram (PPG) from the region 12. The motion artefact sensor 430 may thus be configured to use the laser signal that is used for forming the speckle pattern. Alternatively, the motion artefact sensor may use a separate light signal from a separate light source of the system. The light signal is provided on the region 12 of the person 10 for allowing acquiring of the motion compensation signal.

The motion artefact sensor 430 may comprise a photo-sensitive element for detecting a light intensity transmitted through or reflected by the region 12. Where the facial-cervical region 12 is used, the motion artefact sensor 430 may detect light intensity of reflected light.

The detected light intensity may form a time-varying PPG signal that can be used as a motion compensation signal. The PPG signal may represent a volume of a blood vessel in the region 12. However, the PPG signal may also be affected by motion of the person such that the PPG signal may be used for motion compensation in a similar manner as described above using SPG signals for motion compensation.

The system 400 may thus further comprise a processor 440 configured to receive the sequence of images for determining the wave propagation signal and configured to receive the varying light intensity signal of the PPG for determining the motion compensation signal. This motion compensation signal may be used instead of the motion compensation signal based on SPG described above for providing motion compensation of the first SPG signal representing wave propagation.

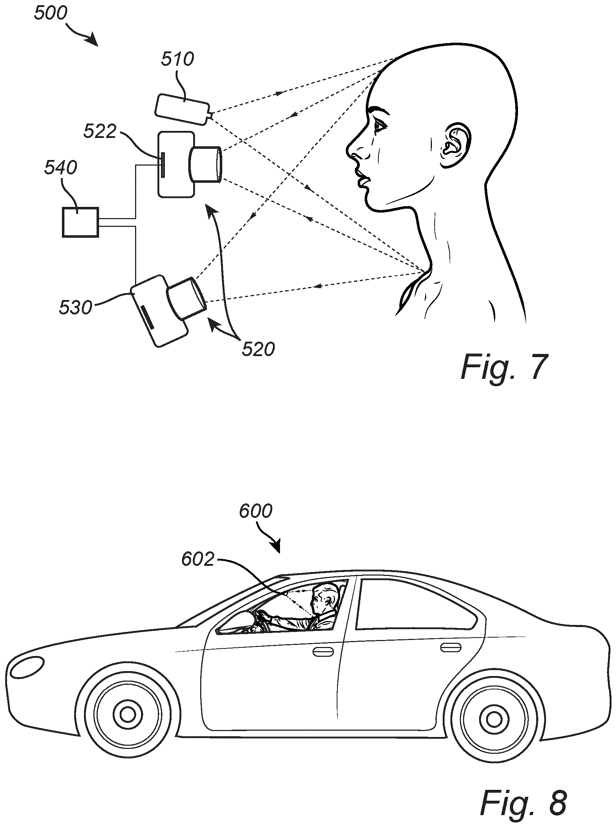

Referring now to Fig. 7, a method and system 500 for motion artefact reduction according to a fourth embodiment will be described.

The system 500 may comprise a laser source 510 as described above. The system 500 may further comprise an imaging unit 520 with a first and a second camera 522, 530. The first camera 522 may corresponding to the imaging unit described above and the second camera 530 of the imaging unit 520 may be used as a motion artefact sensor. Thus, the system 500 may comprise a separate sensor for performing an optical measurement representing the region 12 of the person 10.

The first and second cameras 522, 530 may provide imaging using different magnifications. Each of the first and second cameras 522, 530 may be configured to image at least part of the region 12 of the person 10. The first and second cameras 522, 530 may thus both be configured to acquire sequence of images of the speckle pattern formed on the region 12 of the person 10 but with different magnifications.

Thus, a first SPG signal may be determined from the images acquired with a first magnification using the first camera 522 and a second SPG signal may be determined from the images acquired with a second magnification using the second camera 530.

The different magnifications may imply that the wave propagation and motion artefacts are differently represented in the first and second SPG signals. This implies that the processor 540 may use the second SPG signal for providing motion compensation for the first SPG signal as described above.

It should be further realized that the speckle pattern may be imaged using different magnifications by using a single imaging unit instead of two separate imaging units. For instance, the imaging unit may be provided with a microlens array providing different magnifications for different portions of the image sensor.

As shown in Fig. 8, the system according to any of the above-described embodiments may be implemented in a vehicle 600. A person traveling with the vehicle 600 may be subject to movements, e.g., due to irregularities in a road, acceleration, or deceleration. Thus, a speckle pattern signal acquired in the vehicle 600 may be substantially affected by motion artefacts.

Thus, the vehicle 600 may implement a system 602 according to any one of the above-described embodiments. The system 602 may be mounted for providing acquiring of the speckle pattern signal from a location in which the person 10 will be situated when traveling with the vehicle 600.

The system 602 mounted in the vehicle 600 allows a simple manner of acquiring a motion compensated SPG signal since the system 602 may be set to perform acquisition when the vehicle 600 is used. No activation by the person 10 is needed and no adaptation by the person 10 for allowing acquisition of the motion compensated SPG signal is needed.

In the above the inventive concept has mainly been described with reference to a limited number of examples. However, as is readily appreciated by a person skilled in the art, other examples than the ones disclosed above are equally possible within the scope of the inventive concept, as defined by the appended claims.

**Claims**

1. A method for reducing motion artefacts in a speckle pattern signal, said method comprising:

    illuminating (202) a region of a living being (10) by a light signal to form a speckle pattern;
    acquiring (204) a sequence of images of the region (12) of the living being (10);
    extracting (206) a speckle pattern signal from the acquired sequence of images to form a wave propagation signal representing a variation in time of a pressure wave or volumetric flow wave in the living being (10);
    acquiring (208) a motion compensation signal based on an optical measurement representing the region (12) of the living being (10), wherein the motion compensation signal represents a variation in time of motion of the living being (10); and
    performing (210) motion compensation of the wave propagation signal based on the motion compensation signal.

2. The method according to claim 1, wherein the speckle pattern signal is a first speckle pattern signal, and wherein acquiring of the motion compensation signal is based on determining a second speckle pattern signal representing the speckle pattern to form the motion compensation signal.

3. The method according to claim 2, wherein the first speckle pattern signal is extracted from a first portion (14a) of the region (12) and wherein the second speckle pattern signal is determined from a second portion (14d) of the region (12), wherein the second portion (14d) is different from the first portion (14a).

4. The method according to claim 3, further comprising identifying the first portion (14a) of the region (12) in the sequence of images and the second portion (14b) of the region (12) in the sequence of images based on identifying landmark features in the sequence of images, for using the first portion (14a) of the region (12) in the extracting of the first speckle pattern signal and for using the second portion (14d) of the region (12) in the determining of the second speckle pattern signal.

5. The method according to any one of claims 3-4, wherein acquiring of the motion compensation signal comprises determining one or more further speckle pattern signals from respective further portions (14b, 14c) of the region (12).

6. The method according to claim 5, wherein the motion compensation signal is determined from a combination of the second speckle pattern signal and the one or more further speckle pattern signal.

7. The method according to claim 5, wherein the motion compensation signal is determined from a dynamic selection from the second speckle pattern signal and the one or more further speckle pattern signal.

8. The method according to any one of claims 2-7, wherein the first speckle pattern signal and the second speckle pattern signal are extracted from the sequence of images.

9. The method according to claim 2, wherein the first speckle pattern signal is based on a representation of the speckle pattern with a first lens parameter and the second speckle pattern signal is based on a representation of the speckle pattern with a second lens parameter.

10. The method according to any one of the preceding claims, wherein performing motion compensation comprises forming a linear combination of the wave propagation signal and the motion compensation signal.

11. The method according to any one of the preceding claims, wherein the living being is a human being and the region illuminated by the light signal is at least part of a facial-cervical region.

12. A system (100; 300; 400; 500) for reducing motion artefacts in a speckle pattern signal, the system (100; 300; 400; 500) comprising:

a light source (110; 310; 410; 510) configured to illuminate a region (12) of a living being (10) by a light signal for forming a speckle pattern;
an imaging unit (120; 320; 420; 520) configured to acquire a sequence of images representing the speckle pattern at the region (12) of the living being (10);
a processor (140; 340; 440; 540) configured to:

receive the sequence of images from the imaging unit (120; 320; 420; 520);
extract a speckle pattern signal from the received sequence of images to form a wave propagation signal representing a variation in time of a pressure wave or a volumetric flow wave in the living being (10);
receive a motion compensation signal based on an optical measurement representing the region (12) of the living being (10), wherein the motion compensation signal represents a variation in time of motion of the living being (10); and
perform motion compensation of the wave propagation signal based on the motion compensation signal.

13. The system according to claim 12, wherein the processor (140; 340) is configured to acquire the motion compensation signal based on processing of the received sequence of images.

14. The system according to claim 12, wherein the imaging unit (520) is configured to acquire one or more sequences of images representing the speckle pattern using different lens parameters, wherein the processor (540) is configured to extract the speckle pattern signal and acquire the motion compensation signal based on the different lens parameters.

15. A vehicle (600) comprising:
a system (602) according to any one of claims 12-14, wherein the system (602) is mounted in the vehicle (600) for illuminating the living being that is a human being traveling with the vehicle (600), wherein the system (602) is configured to determine a motion compensated wave propagation signal compensating for motion artefacts due to movement of the vehicle (600).

Fig. 1

| Illuminating a region of a living being | 202 |

| Acquiring a sequence of the region | 204 |

| Extracting a speckle pattern signal from the acquired sequence of images to form a wave propagation signal | 206 |

| Acquiring a motion compensation signal | 208 |

| Performing motion compensation of the wave propagation signal based on the motion compensation signal | 210 |

Fig. 2

EP 4 763 064 A1

14d — Forehead

12

Eyes

14c —

14b — Chin

14a — Neck

214
Get 5 s of SPG_neck and SPG_forehead

214
Signal normalization

216
Subtract SPGs SPG_neck - SPG_forehead

22014
Combine segments for time domain signal (overlap-add procedure)

222
Slide of 1 second

*Fig. 3*

Fig. 4

Fig. 5

Fig. 6

500

510

522

540

530

520

Fig. 7

600

602

Fig. 8

Europäisches
Patentamt

European
Patent Office

Office européen
des brevets

## EUROPEAN SEARCH REPORT

**Application Number**

EP 24 22 1640

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2023/208751 A1 (TRINAMIX GMBH [DE]) 2 November 2023 (2023-11-02) | 1,10-13, 15 | INV. A61B5/024 |
| A | * page 3, line 14 - page 4, line 4 *<br>* page 22, lines 18-23 *<br>* page 23, lines 8-14 *<br>* page 25, lines 16-17 *<br>* page 32, line 2 - page 33, line 9 *<br>* abstract; figures 1-7 * | 2-9,14 | A61B5/026 G02B27/48 |
| X | US 2023/380708 A1 (ANDRÉ MARC [CH] ET AL) 30 November 2023 (2023-11-30) | 1,2,8, 10,12,13 | |
| A | * abstract; figures 7,18,19 *<br>* paragraphs [0003], [0021], [0045], [0049], [0051], [0146], [0148] - [0150] * | 3-7,9, 14,15 | |
| X | WO 2017/045976 A1 (KONINKLIJKE PHILIPS NV [NL]) 23 March 2017 (2017-03-23) | 1,10-13 | |
| A | * abstract; figures 2-5 *<br>* page 7, lines 4-10 *<br>* page 8, lines 3-13 *<br>* page 16, lines 22-29 *<br>* page 19, lines 19-25 * | 2-9,14, 15 | |
| | | | **TECHNICAL FIELDS SEARCHED (IPC)** |
| | | | A61B G02B |
| A | US 2022/039679 A1 (CALIFA RAN [IL] ET AL) 10 February 2022 (2022-02-10) * abstract; figure 9 * * paragraph [0081] * | 1-15 | |

The present search report has been drawn up for all claims

| Place of search | Date of completion of the search | Examiner |
|---|---|---|
| Berlin | 21 May 2025 | Sleightholme, G |

EPO FORM 1503 03.82 (P04C01)

## ANNEX TO THE EUROPEAN SEARCH REPORT
## ON EUROPEAN PATENT APPLICATION NO.

EP 24 22 1640

This annex lists the patent family members relating to the patent documents cited in the above-mentioned European search report.
The members are as contained in the European Patent Office EDP file on
The European Patent Office is in no way liable for these particulars which are merely given for the purpose of information.

21-05-2025

| Patent document cited in search report | Publication date | Patent family member(s) | | Publication date |
|---|---|---|---|---|
| WO 2023208751 A1 | 02-11-2023 | CN | 119136733 A | 13-12-2024 |
| | | EP | 4514216 A1 | 05-03-2025 |
| | | KR | 20250003629 A | 07-01-2025 |
| | | WO | 2023208751 A1 | 02-11-2023 |
| US 2023380708 A1 | 30-11-2023 | EP | 4084692 A1 | 09-11-2022 |
| | | US | 2021201444 A1 | 01-07-2021 |
| | | US | 2023380708 A1 | 30-11-2023 |
| | | WO | 2021134130 A1 | 08-07-2021 |
| WO 2017045976 A1 | 23-03-2017 | NONE | | |
| US 2022039679 A1 | 10-02-2022 | EP | 3886686 A1 | 06-10-2021 |
| | | JP | 2022508237 A | 19-01-2022 |
| | | US | 2022039679 A1 | 10-02-2022 |
| | | WO | 2020110116 A1 | 04-06-2020 |

EPO FORM P0459

For more details about this annex : see Official Journal of the European Patent Office, No. 12/82